# EUROPÄISCHE PATENTSCHRIFT

(11) **EP 0 666 073 B1**
(45) Veröffentlichungstag und Bekanntmachung des Hinweises auf die Patenterteilung: **07.05.1997**
(21) Anmeldenummer: 94119389.8
(22) Anmeldetag: 08.12.1994
(51) Int. Cl.: A61K 7/09

(54) **Mittel zur dauerhaften Verformung von menschlichen Haaren**
Permanent human hair waving composition
Composition pour la mise en plis permanente des cheveux humains

(30) Priorität: 27.01.1994 DE 4402328
(43) Veröffentlichungstag der Anmeldung: 09.08.1995
(73) Patentinhaber: GOLDWELL GmbH, D-64297 Darmstadt (DE)
(72) Erfinder: Glauder, Jan, Dr., 64297 Darmstadt (DE)

(56) Entgegenhaltungen:
- EP-A- 0 235 783
- EP-A- 0 237 870
- EP-A- 0 344 653
- EP-A- 0 363 057
- EP-A- 0 551 135
- WO-A-89/05627
- DE-A- 3 620 849
- DE-A- 4 306 915
- DE-C- 4 109 365
- FR-A- 1 076 766

## Beschreibung

Die vorliegende Erfindung betrifft ein Mittel zum Verformen von menschlichen Haaren, d.h. ein Dauerwellmittel, das eine ausgezeichnete Wellwirkung besitzt, jedoch auf das Haar auch bei mehrfacher Anwendung keinerlei schädigende Wirkung ausübt.

Die Dauerwellung erfordert bekanntlich zwei Behandlungsschritte: Die reduktive Spaltung der Cystin-Disulfidbrücken des Haares durch Einwirkung eines Reduktionsmittels und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch Thioglykolsäure, insbesondere als Ammoniumsalz, obwohl zahlreiche andere Thio-Verbindungen für diesen Zweck vorgeschlagen wurden, die sich jedoch in der Praxis zumeist nicht durchgesetzt haben.

Die Thioglykolat enthaltenden Zusammensetzungen werden üblicherweise bei einem pH-Wert zwischen 8 und 10, insbesondere 8,5 bis 9,5, eingesetzt, was bei wiederholter, zeitlich nahe zusammenliegender Anwendung zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h. um den Neutralpunkt herum, liegt. Der in diesen Zusammensetzungen meistbenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirkt, so daß auch diese Lösung nicht optimal ist.

Es wurde nunmehr gefunden, daß diese Probleme dadurch überwunden werden können und Dauerwellmittel, die nicht haarschädigend sind, jedoch gleichwohl eine gute Wellwirkung aufweisen, erhalten werden, wenn man ein Mittel einsetzt, daß mindestens eine reduzierend wirkende Thioverbindung, vorzugsweise Thioglykolsäure und/oder Thiomilchsäure bzw. deren Salze, sowie eine Kombination aus
a) etwa 0,25 bis etwa 5 Gew.-% Ethylalkohol, n-Propylalkohol und/oder Isopropylalkohol;
b) etwa 0,25 bis etwa 2,5 Gew.-% mindestens einer Komponente aus der Gruppe 1,2-Propandiol, 1,2-Butandiol, 1,3-Butandiol, Glycerin und/oder Dipropylenglykolmonomethylether; und
c) etwa 0,25 bis etwa 7,5 Gew.-% 1-Methoxypropanol(-2) und/oder Diethylenglykolmonomethyl- bzw. -monoethylether, jeweils berechnet auf die Gesamtzusammensetzung des gebrauchsfertigen Mittels, enthält.

Gemäß einer bevorzugten Ausführungsform der Erfindung weist die gebrauchsfertige, zum Aufbringen auf das Haar bestimmte Dauerwell-Zusammensetzung einen pH-Wert zwischen etwa 7 und 8,5 auf.

Wie bereits dargestellt, enthält das erfindungsgemäße Dauerwellmittel als bevorzugte reduzierend wirkende Thioverbindungen Thioglykolsäure und/oder 2- bzw. 3-Thiomilchsäure bzw. deren Salze, beispielsweise Diammonium- bzw. Alkalisalze.

Es können jedoch auch andere wellwirksame Thioverbindungen mitverwendet werden, beispielsweise die aus der EP-A 235 783 bekannten Thioglyceryl-C₁-C₂₀-alkylether oder 1-Phenyl-2-mercaptoethanol sowie die in der EP-A 461 526 beschriebenen C₁-C₂₀-Alkoxyethoxythioglycerylether.

Der Mengenanteil der zum Einsatz gelangenden Thioverbindungen mit Reduktionswirkung ist von deren Struktur abhängig und liegt im allgemeinen zwischen etwa 2,5 und etwa 25 Gew.-% der gebrauchsfertigen Zusammensetzung, vorzugsweise, insbesondere wenn Thioglykolsäure oder Thiomilchsäure verwendet werden, zwischen etwa 3 und etwa 15 Gew.-%.

Auch die Verwendung anorganischer Sulfite, beispielsweise von Natriumbisulfit, ist grundsätzlich möglich.

Neben den genannten und anderen, an sich bekannten Reduktionsmitteln enthalten Dauerwell-Zusammensetzungen üblicherweise ein Alkalisierungsmittel, dessen Konzentration, in Abhängigkeit von Art und Menge der reduzierenden Thioverbindungen, zwischen etwa 1 bis etwa 10, insbesondere etwa 2 bis etwa 8 Gew.-%, der gebrauchsfertigen Zusammensetzung liegt.

Bevorzugte Alkalisierungsmittel im Rahmen der Erfindung sind Ammoniumhydrogencarbonat, Ammoniumcarbonat und Ammoniumcarbamat.

Das Alkalisierungsmittel kann gemeinsam mit der wellwirksamen Thioverbindung in einer Phase vorliegen; es kann jedoch, aus Kompatibilitäts- und Stabilitätsgründen, auch sinnvoll sein, beide Stoffe in jeweils bis zur Anwendung getrennt gehaltener Form, beispielsweise in Zweikammerbehältern, zu lagern, die erst unmittelbar vor der Applikation auf das Haar vermischt werden.

Die zweite essentielle erfindungsgemäße Substanz, mindestens eine Verbindung aus der Gruppe Ethylalkohol, n-Propylalkohol und/oder Isopropylalkohol, ist in einer Menge von 0,25 bis etwa 5 Gew.-%, vorzugsweise 0,5 bis 1,5 Gew.-%, jeweils berechnet auf die Gesamtzusammensetzung, enthalten. Vorzugsweise wird Isopropylalkohol eingesetzt.

Der dritte essentielle Bestandteil, mindestens eine Komponente aus der Gruppe 1,2-Propandiol, 1,2-Butandiol, 1,3-Butandiol, Glycerin und/oder Dipropylenglykolmonomethylether, liegt in einer Menge von 0,25 bis etwa 2,5 Gew.-%, berechnet auf die gebrauchsfertige Gesamtzusammensetzung, vor. Bevorzugt ist 1,2-Propandiol in einer Menge von etwa 0,5 bis etwa 2 Gew.-%.

Der letzte essentielle erfindungsgemäße Bestandteil, 1-Methoxypropanol(-2) und/oder Diethylenglykolmonomethyl- bzw. -monoethylether, ist in einer Menge zwischen etwa 0,25 und etwa 7,5 Gew.%, berechnet auf die gebrauchsfertige Gesamtzusammensetzung, enthalten. Hier ist Diethylenglykolmonoethylether, auch unter dem Namen Ethoxydiglykol bzw. Ethylcarbitol bekannt, in einer Menge zwischen etwa 1 und etwa 5 Gew.-% bevorzugt.

Die erfindungsgemäßen Dauerwellmittel können alle für diesen Zweck vorgeschlagenen und eingesetzten Zusatzstoffe enthalten.

Zur Vermeidung von Wiederholungen wird hierzu auf den einschlägig bekannten Stand der Technik und dessen Zusammenfassungen, beispielsweise in "Ullmann's Encyclopaedia of Industrial Chemistry", Vol.A12 (1986), S.588 bis 591, sowie die Monographie von K.Schrader, "Grundlagen und Rezepturen der Kosmetika", 2. Auflage (1989, Hüthig Buchverlag), S.823 bis 840, verwiesen.

Falls das Mittel in zwei bis zur Anwendung getrennten Phasen vorliegt, ist es vorteilhaft, den pH-Wert einer Phase im sauren Bereich zwischen etwa 4,5 und 6,5 einzustellen.

Dies geschieht vorzugsweise durch Zusatz eines Aminosäurehydrochlorids, insbesondere Cysteinhydrochlorid, wodurch auch noch eine zusätzliche Verstärkung der Wellwirkung erzielt werden kann, und Glycinhydrochlorid.

Es können jedoch auch weitere Aminosäurehydrochloride, beispielsweise die des Alanins, Valins, Leucins, Isoleucins, Prolins, Tryptophans, Phenylalanins, Methionins, Serins, Tyrosins, Threonins, Asparagins, Glutamins oder Histidins, enthalten sein.

Der Anteil des Aminosäurehydrochlorids wird bestimmt durch die Einstellung des erforderlichen pH-Wertes; der Mindestanteil liegt bei etwa 0,5 Gew.-%, der Höchstanteil bei etwa 5 Gew.-%, bezogen auf reine Aminosäure und die Gesamtzusammensetzung des Mittels (also die Summe der bis zur Anwendung getrennt gehaltenen Zusammensetzungen).

Bei solchen Zweikomponenten-Präparaten weist dann die das alkalisierende Ammoniumsalz enthaltende Zusammensetzung einen alkalischen pH-Wert, vorzugsweise im Bereich zwischen etwa 8 und 9,5, auf.

Die Fixierung der durch Anwendung der erfindungsgemäßen Dauerwell-Zusammensetzungen verformten Haare erfolgt durch die ebenfalls bekannte und übliche Neutralisation mittels Wasserstoffperoxid oder Alkalibromaten.

Falls die erfindungsgemäßen Zusammensetzungen in zwei bis zur Anwendung voneinander getrennten Phasen vorliegen, erfolgt die getrennte Unterbringung der beiden Zusammensetzungen bis zur Anwendung des Mittels vorzugsweise in den seit langem bekannten Zweikammerbehältern, deren Trennwand bei der Anwendung durch geeignete Mittel zerstört wird.

Solche Zweikammerbehältnisse, die sich insbesondere für die erfindungsgemäßen Zusammensetzungen eignen, sind beispielsweise in den deutschen Offenlegungsschriften 35 28 525, 36 06 003, 38 12 343 und 38 37 595 beschrieben.

Die folgenden Beispiele dienen der Illustration der Erfindung.

### Beispiel 1

| Zusammensetzung A: | |
|---|---|
| Ammoniumhydrogencarbonat | 4,5 (g) |
| Kationisches Polymer | 0,7 |
| Nichtionischer Lösungsvermittler (Ethylenoxid-Kondensat) | 0,8 |
| Isopropylalkohol | 1,5 |
| Diethylenglykolmonoethylether | 2,0 |
| Trübungsmittel, Parfum | q.s. |
| Ammoniak, 25%ig | @ pH 8,4 |
| Wasser | @ 72,2 |

| Zusammensetzung B: | |
|---|---|
| Ammoniumthioglykolat, 70%ig | 18,5 (g) |
| 2-Thiomilchsäure | 2,0 |
| Cysteinhydrochlorid x H₂O | 3,0 |
| 1,2-Propandiol | 0,5 |
| Ammoniak, 25%ig | @ pH 5,55 |
| Wasser | @ 27,8 |

65 g der Zusammensetzung A und 25 g der Zusammensetzung B wurden getrennt in eine übliche Zweikammerdose verpackt.

Beim Zusammenbringen beider Zusammensetzungen wurde ein Produkt mit einem pH-Wert von 7,45 erhalten, das bei der Anwendung in einer üblichen Dauerwellbehandlung mit anschließender Peroxid-Fixierung eine exzellent ausgebildete, stabile Dauerwellung ohne jedwede Haarschädigung erzielte.

### Beispiel 2

| Zusammensetzung A: | |
|---|---|
| Ammoniumhydrogencarbonat | 4,5 (g) |
| Nichtionogener Lösungsvermittler (Ethoxlat) | 1,0 |
| Parfum | 0,3 |
| Ethanol | 0,5 |
| Diethylenglykolmonoethylether | 1,0 |
| Ammoniak, 25%ig | @ pH 8,4 |
| Wasser | @ 72,2 |

| Zusammensetzung B: | |
|---|---|
| Ammoniumthioglykolat, 70%ig | 18,5 (g) |
| Cysteinhydrochlorid x H₂O | 3,0 |
| Dipropylenglykolmonomethylether | 1,5 |
| Ammoniak, 25%ig | @ pH 5,55 |
| Wasser | @ 27,8 |

Die Verpackung und Applikation der Zusammensetzungen A und B erfolgte wie in Beispiel 1 beschrieben.

Die anwendungsfertige Gesamtmischung wies einen pH-Wert von 7,5 auf.

### Beispiel 3

| Zusammensetzung A: | |
|---|---|
| Ammoniumhydrogencarbonat | 3,0 (g) |
| Nichtionogener Lösungsvermittler (Ethoxylierungs-Produkt) | 1,0 |
| Parfum | 0,5 |
| Isopropylalkohol | 0,5 |
| 1-Methoxypropanol(-2) | 4,0 |
| Ammoniak, 25%ig | @ pH 8,4 |
| Wasser | @ 72,2 |

| Zusammensetzung B: | |
|---|---|
| Ammonium-2-thiolactat, 50%ig | 20,0 (g) |
| Cysteinhydrochlorid x H₂O | 1,5 |
| 1,2-Propandiol | 0,5 |
| Ammoniak, 25%ig | @ pH 5,55 |
| Wasser | @ 27,8 |

Die Abpackung und Anwendung erfolgte wie in Beispiel 1 beschrieben; die gebrauchsfertige Mischung wies einen pH-Wert von 7,4 auf.

### Beispiel 4

| | |
|---|---|
| Ammoniumcarbonat | 0,5 (g) |
| Ammoniumhydrogencarbonat | 3,0 |
| Ammoniumthioglykolat, 70%ig | 14,5 |
| Cysteinhydrochlorid x H₂O | 3,0 |
| Nichtionogener Lösungsvermittler | 1,0 |
| Parfum | 0,3 |
| Isopropylalkohol | 0,5 |
| Diethylenglykolmonoethylether | 4,0 |
| 1,2-Propandiol | 1,0 |
| Ammoniak, 25%ig | @ pH 8,0 |
| Wasser | @ 100,0 |

Auch mit dieser Zusammensetzung wurde, nach Peroxid-Fixierung, eine ausdrucksvolle Dauerwellung erhalten; auch bei mehrfach aufeinanderfolgender Anwendung war keinerlei Haarschädigung festzustellen.

### Beispiel 5

| | |
|---|---|
| Ammoniumcarbamat | 2,0 (g) |
| Ammonium-2-thiolactat, 50%ig | 4,0 |
| Ammoniumthioglykolat, 50%ig | 20,8 |
| Cysteinhydrochlorid x H₂O | 5,0 |
| Nichtionogener Lösungsvermittler | 1,0 |
| Parfum | 0,3 |
| n-Propanol | 1,5 |
| 1-Methoxypropanol(-2) | 0,5 |
| 1,3-Butandiol | 2,0 |
| Ammoniak, 25%ig | @ pH 8,4 |
| Wasser | @ 100,0 |

Es wurde ein ähnlich gutes Dauerwellergebnis wie mit der in Beispiel 4 beschriebenen Zusammensetzung erhalten.

## Patentansprüche

1. Mittel zur dauerhaften Verformung von menschlichen Haaren, enthaltend mindestens eine reduzierend wirkende Thioverbindung und eine Kombination aus
a) etwa 0,25 bis etwa 5 Gew.-% Ethylalkohol, n-Propylalkohol und/oder Isopropylalkohol;
b) etwa 0,25 bis etwa 2,5 Gew.-% mindestens einer Komponente aus der Gruppe 1,2-Propandiol, 1,2-Butandiol, 1,3-Butandiol, Glycerin und/oder Dipropylenglykolmonomethylether, und
c) etwa 0,25 bis etwa 7,5 Gew.-% 1-Methoxypropanol(-2) und oder Diethylenglykolmonomethyl- bzw. -monoethylether, jeweils berechnet auf die Gesamtzusammensetzung des gebrauchsfertigen Mittels.

2. Mittel nach Anspruch 1, dadurch gekennzeichnet, daß die gebrauchsfertige, zum Aufbringen auf das Haar bestimmte Zusammensetzung einen pH-Wert zwischen etwa 7 und etwa 8,5 aufweist.

3. Mittel nach Anspruch 1 oder 2, dadurch gekennzeichnet, daß es als reduzierend wirkende Thioverbindung Thioglykolsäure und/oder 2-Thiomilchsäure bzw. deren Salze enthält.

4. Mittel nach einem oder mehreren der vorhergehenden Ansprüche, dadurch gekennzeichnet, daß es 0,5 bis 2,0 Gew.-% 1,2-Propandiol enthält.

5. Mittel nach einem oder mehreren der vorhergehenden Ansprüche dadurch gekennzeichnet, daß es etwa 1 bis 5 Gew.-% Diethylenglykolmonoethylether enthält.

6. Mittel nach einem oder mehreren der vorhergehenden Ansprüche, dadurch gekennzeichnet, daß es etwa 0,5 bis 5 Gew.-% Ammoniumhydrogenarbonat, Ammoniumcarbonat und/oder Ammoniumcarbamat enthält.

7. Mittel nach einem oder mehreren der vorhergehenden Ansprüche, dadurch gekennzeichnet, daß es aus zwei bis zur Anwendung voneinander getrennt gehaltenen Zusammensetzungen besteht, wobei die eine Zusammensetzung (A) mindestens ein Aminosäuresalz in wäßrigem Medium enthält und auf einen pH-wert zwischen etwa 4,5 und 6,5 eingestellt ist, und die zweite, davon getrennt gehaltene Zusammensetzung (B) Ammoniumhydrogencarbonat, Ammoniumcarbonat und/oder Ammoniumcarbamat enthält und einen pH-Wert zwischen etwa 8 und 9,5 aufweist, und beide Zusammensetzungen unmittelbar vor der Anwendung auf menschliches Haar vermischt werden und die dadurch hergestellte gebrauchsfertige Zusammensetzung (AB) einen pH-Wert zwischen etwa 7 und 8 aufweist.

8. Mittel nach Anspruch 7, dadurch gekennzeichnet, daß die reduzierende Thioverbindung zumindest teilweise in der Zusammensetzung (A) enthalten ist.

## Claims

1. Composition for permanent waving of human hair comprising at least one active reducing thio compound and a combination of
a) about 0.25% to about 5% by wt. of ethyl alcohol , n-propyl alcohol, and (or) isopropyl alcohol;
b) about 0.25% to about 2.5% by wt. of at least one compound selected from the group of 1,2-propanediol, 1,2-butanediol, 1,3-butanediol, glycerol and (or) dipropyleneglycol monomethylether, and
c) about 0.25% to about 7.5% by wt. of 1-methoxypropanol(-2) and (or) diethyleneglycol monomethyl- or monoethylether,
each calculated to the total composition of the ready-to-use preparation.

2. Composition according to claim 1, characterized in that the ready-to-use composition has a pH-value from about 7 to about 8.5.

3. Composition according to claim 1 or 2, characterized in that it contains thioglycolic acid and (or) 2-thiolactic acid or the salts thereof as the active reducing thio compound.

4. Composition according to one or more of the preceding claims, characterized in that it contains 0.5% to 2.0% by wt. of 1,2-propanediol.

5. Composition according to one or more of the preceding claims, characterized in that it contains about 1% to about 5% by wt. of diethyleneglycol monoethylether.

6. Composition according to one or more of the preceding claims, characterized in that it contains about 0.5% to 5% by wt. of ammonium hydrogen carbonate, ammonium carbonate and (or) ammonium carbamate.

7. Composition according to one or more of the preceding claimns, characterized in that it consists of two compositions which are kept separate until application, whereof one composition (A) comprises at least one amino acid salt in an aqueous medium having a pH-value between about 4.5 to 6.5, and the second composition (B), which is kept separate from composition (A), comprises ammonium hydrogen carbonate, ammonium carbonate and (or) ammonium carbamate and has a pH-value between about 8 and 9.5, which two compositions are admixed immediately before application onto human hair, whereby the ready-to-use composition (AB) obtained thereby has a pH-value between about 7 and 8.

8. Composition according to claim 7, characterized in that the reducing thio compound is at least partly contained in composition (A).

## Revendications

1. Agent de mise en forme permanente des cheveux humains, contenant au moins un composé thio agissant comme réducteur et une combinaison de
a) environ 0,25 à environ 5 % en poids d'alcool éthylique, d'alcool n-propylique et/ou d'alcool isopropylique;
b) environ 0,25 à environ 2,5 % en poids au moins d'un composant du groupe du propane-1,2-diol, du butane-1,2-diol, du butane-1,3-diol, de la glycérine et/ou de l'éther monométhylique du dipropylèneglycol, et
c) environ 0,25 à environ 7,5 % en poids de 1-méthoxypropan-2-ol et/ou d'éther monométhylique, ou d'éther monoéthylique du diéthylèneglycol, calculés à chaque fois par rapport à la composition globale de l'agent prêt à l'emploi.

2. Agent selon la revendication 1, caractérisé en ce que la composition prête à l'emploi, destinée à l'application sur la chevelure, présente une valeur de pH comprise entre environ 7 et environ 8,5.

3. Agent selon la revendication 1 ou 2, caractérisé en ce qu'il contient, en tant que composé thio agissant comme réducteur, de l'acide thioglycolique et/ou de l'acide 2-thiolactique, ou leurs sels.

4. Agent selon l'une ou plusieurs des revendications précédentes, caractérisé en ce qu'il contient de 0,5 à 2,0 % en poids de propane-1,2-diol.

5. Agent selon l'une ou plusieurs des revendications précédentes, caractérisé en ce qu'il contient environ de 1 à 5 % en poids d'éther monoéthylique du diéthylèneglycol.

6. Agent selon l'une ou plusieurs des revendications précédentes, caractérisé en ce qu'il contient environ de 0,5 à 5 % en poids d'hydrogénocarbonate d'ammonium, de carbonate d'ammonium et/ou de carbamate d'ammonium.

7. Agent selon l'une ou plusieurs des revendications précédentes, caractérisé en ce qu'il se compose de deux compositions maintenues séparées l'une de l'autre jusqu'à utilisation, ladite composition (A) contenant au moins un sel d'acide aminé en milieu aqueux et étant ajustée à une valeur de pH comprise entre environ 4,5 et 6,5, et ladite deuxième composition (B), maintenue séparée de cette dernière, contenant de l'hydrogénocarbonate d'ammonium, du carbonate d'ammonium et/ou du carbamate d'ammonium et présentant une valeur de pH comprise entre environ 8 et 9,5, et les deux compositions étant mélangées immédiatement avant utilisation sur la chevelure humaine, et la composition (AB), prête à l'emploi ainsi préparée, présentant une valeur de pH comprise entre environ 7 et 8.

8. Agent selon la revendication 7, caractérisé en ce que la composition thio réductrice est contenue au moins en partie dans la composition (A).
